# DEMANDE DE BREVET EUROPEEN

(11) **EP 1 070 508 A1**
(43) Date de publication de la demande: **24.01.2001**
(21) Numéro de dépôt: 00420164.6
(22) Date de dépôt: 21.07.2000
(51) Int. Cl.: A61L 27/34

(54) **Objet ayant une surface extérieure anti-adhérente vis à vis des microorganismes**

(30) Priorité: 01.12.1999 FR 9915164; 21.07.1999 FR 9909624
(71) Demandeur: Institut National des Sciences Appliquées de Lyon, 69621 Villeurbanne (FR); Association pour les transferts de technologie Du Mans, 72000 Le Mans (FR)
(72) Inventeur: Le-Thi, Thanh-Thuy, 69005 Lyon (FR); Lejeune, Philippe, 69100 Villeurbanne (FR); Legeay, Gilbert, 72650 Saint-Saturnin (FR)
(74) Mandataire: Maureau, Philippe

(57) **Abrégé**

Utilisation en tant qu'objet ou matériau dont la surface extérieure est anti-adhérente vis à vis de micro-organismes, par exemple des bactéries, et/ou vis à vis d'entités biologiques comportant des protéines, d'un objet ou matériau susceptible d'être obtenu par le procédé suivant:
a) On part d'undit objet ou matériau comprenant un substrat ayant une surface exposée ;
b) On effectue un greffage chimique en une seule étape pendant laquelle concomitamment, d'une part on met la surface exposée dudit objet ou matériau, en contact avec une atmosphère gazeuse comprenant au moins une espèce chimique, susceptible d'être liée chimiquement audit substrat au niveau de sa surface exposée, sans se polymériser, et apte à former un groupement polaire une fois liée audit substrat, et d'autre part on active l'espèce chimique et/ou la surface exposée, par apport d'énergie in situ, par exemple rayonnée ;
c) Selon une étape de couchage, on dépose au moins un matériau polymère hydrosoluble sur la surface exposée greffée, et ledit polymère hydrosoluble est lié par liaison faible, par exemple par liaison hydrogène, à la surface exposée greffée, à l'exclusion de pratiquement toute liaison chimique, d'une part entre ledit matériau polymère et la surface greffée exposée, et d'autre part au sein dudit matériau polymère, par réticulation.

## Description

La présente invention concerne de manière générale le traitement d'un objet ou matériau, comprenant un substrat dont toute surface exposée, par exemple extérieure ou intérieure, est susceptible d'entrer en contact avec un milieu biologique, liquide ou solide, comportant des bactéries et/ou des entités biologiques comportant des protéines, ce traitement ayant pour objectif principal de rendre ladite surface biocompatible.

Conformément au document EP-A-0 611 792, on a proposé un procédé de traitement d'un substrat, en vue de le rendre biocompatible, selon lequel, successivement on effectue une étape de greffage chimique, pour obtenir une surface exposée sur laquelle ont été greffés des groupements polaires, puis une étape de couchage, pour déposer ou lier au moins un matériau polymère sur la surface exposée greffée.

L'étape de greffage chimique est effectuée en plusieurs opérations, à savoir :
- une première opération, pendant laquelle concomitamment, d'une part on met la surface exposée de l'objet ou du matériau en contact avec une atmosphère gazeuse comprenant de l'oxygène, et d'autre part on active l'oxygène, et/ou la surface exposée, par apport d'énergie in situ, par exemple rayonnée
- une deuxième opération pendant laquelle on applique sur la surface exposée, traitée selon la première opération, une solution aqueuse comprenant un monomère acrylamide et des ions cériques, moyennant quoi ledit monomère est greffé sur la surface exposée
- et une troisième opération, par laquelle on lie chimiquement des groupements polaires, à savoir amine ou carboxyle, sur les monomères acrylamides, moyennant quoi on obtient une surface exposée sur laquelle ont été greffés indirectement des groupements polaires.

L'étape de couchage est effectuée avec des matériaux biologiques, ou biofonctionnels, dont certains sont des macromolécules ou polymères naturels, essentiellement sous forme d'hydrogels. Cette étape est effectuée, soit par liaison chimique, soit par liaison ionique.

L'objet ou matériau ainsi traité et obtenu ne présente pas une biocompatibilité assurée, en raison des étapes, purement chimiques que constituent les deuxième et troisième opérations de l'étape de greffage chimique, et éventuellement l'étape de couchage, qui peuvent laisser subsister des résidus, tels que solvants ou autres, toxiques.

Conformément au document EP-A-0 611 576, on a proposé un procédé de traitement d'un outil à usage médical, selon lequel :
(a) selon une étape de greffage chimique, on met la surface exposée de l'outil en contact avec un plasma comprenant une espèce chimique susceptible d'être liée chimiquement au substrat de l'outil, au niveau de sa surface exposée, et apte à former un groupement polaire, préférentiellement carboxy, par exemple anhydride d'acide, une fois liée audit substrat, moyennant quoi on obtient ladite surface exposée sur laquelle lesdits groupements polaires sont greffés
(b) selon une étape de couchage, on dépose et lie un polymère hydrosoluble à la surface exposée greffée de l'outil :
   - en disposant de ou préparant un polymère hydrosoluble modifié, comportant des restes réactifs, à savoir époxy, halogénure, ou isocyanate
   - en faisant réagir chimiquement le polymère hydrosoluble modifié avec la surface exposée greffée de l'outil
   - et en réticulant le polymère hydrosoluble modifié, lié chimiquement avec la surface exposée greffée de l'outil.

Un tel procédé permet sans doute d'obtenir une biocompatibilité suffisante pour un contact très limité ou très ponctuel, par exemple avec un tissu vivant. Il ne permet pas d'assurer une biocompatibilité à un matériau ou objet susceptible de rester plus longtemps en contact avec tout milieu biologique, en raison des opérations chimiques requises, au minimum pour lier le polymère hydrosoluble à la surface exposée, et réticuler le polymère hydrosoluble lié. Comme précédemment, ces opérations chimiques sont susceptibles de laisser subsister des résidus toxiques dans le biomatériau finalement obtenu.

Conformément au document FR-A-2 620 624, on a décrit un procédé de traitement d'un objet ou matériau, pour lui conférer non seulement une biocompatibilité, mais surtout une forte hémocompatibilité et une faible thrombogénéité. Ce procédé consiste essentiellement en un greffage chimique, selon lequel on met une surface exposée de l'objet ou du matériau, en contact avec un plasma comprenant une espèce chimique fluorée susceptible d'être liée chimiquement au substrat de l'objet ou du matériau, au niveau de sa surface exposée, et apte à former un groupement fluoré, à savoir F, CF₂, ou CF₃, une fois liée audit substrat, moyennant quoi on obtient ladite surface exposée sur laquelle sont greffés lesdits groupements fluorés.

Un tel procédé ne permet pas d'obtenir des objets ou matériaux, dont la surface exposée présente une faible adhérence vis-à-vis de tout milieu biologique entrant à son contact, comportant des micro-organismes, par exemple des bactéries et/ou des entités biologiques comportant des protéines.

Le document WO-A-96/23602 décrit, afin d'améliorer ses propriétés de glissement, le revêtement d'un substrat comportant des groupements amine, par deux couchages successifs, à savoir un premier couchage constitué exclusivement par un polymère de type polyuréthane-polyurée avec des groupes isocyanate réactifs, lesquels réagissent de manière covalente avec les groupements amine du substrat, et une deuxième couchage constitué par un polymère hydrophile.

Le document WO-A-98/58690 décrit, afin d'améliorer ses propriétés de glissement, le revêtement d'un substrat, en couchant sur ce dernier un mélange d'un polymère réticulable et d'un polymère hydrophile, avec un solvant, par exemple le toluène. Un tel mélange a une certaine toxicité, et ne peut conduire à un revêtement biocompatible.

Le document WO-A-94/06485 décrit un traitement hydrophilisant de surface d'un substrat, par greffage chimique de certains composés, par exemple un carbohydrate, sur des groupements chimiques appartenant au substrat, en présence de certains agents chimiques comme NalO₄, NaCNBH₃, etc ... Un tel greffage chimique laisse nécessairement subsister des résidus toxiques.

La publication de C. Baquey et al., Nuclear Instruments and Methods in Physics Research, Section-B : Beam Interactions with Materials and Atoms, NL, North Holland Publishing Company, Amsterdam, décrit l'obtention d'un tissu à base d'un polymère, rendu adhérent à des cellules et protéines, par le greffage d'oligopeptides.

La présente invention a pour objet un matériau ou objet, non seulement biocompatible, mais également faiblement ou peu adhérent vis à vis de tout milieu biologique entrant à son contact, comportant des micro-organismes, par exemple des bactéries, et/ou des entités biologiques comportant des protéines, et ce quel que soit le substrat utilisé.

Selon la présente invention, on obtient un objet ou matériau dont la surface extérieure est anti-adhérente, par utilisation d'un objet ou matériau susceptible d'être obtenu par le procédé suivant :
a) on part d'undit objet ou matériau comprenant un substrat ayant une surface exposée,
b) on effectue un greffage chimique en une seule étape pendant laquelle concomitamment, d'une part on met la surface exposée dudit objet ou matériau, en contact avec une atmosphère gazeuse comprenant au moins une espèce chimique, susceptible d'être liée chimiquement audit substrat au niveau de sa surface exposée, sans se polymériser, et apte à former un groupement polaire une fois liée audit substrat, et d'autre part on active l'espèce chimique et/ou la surface exposée, par apport d'énergie in situ, par exemple rayonnée ,
c) selon une étape de couchage, on dépose au moins un matériau polymère hydrosoluble sur la surface exposée greffée, et ledit polymère hydrosoluble est lié par liaison faible, par exemple par liaison hydrogène, à la surface exposée greffée, à l'exclusion de pratiquement toute liaison chimique, d'une part entre ledit matériau polymère et la surface greffée exposée, et d'autre part au sein dudit matériau polymère, par réticulation.

L'intérêt d'un biomatériau présentant une faible adhérence biologique, c'est-à-dire une faible aptitude à adhérer à différents micro-organismes, par exemple bactéries, n'est pas à démontrer. En effet, on connaît les propriétés d'adhésion bactérienne et protéique de nombreux biomatériaux, tels que silicones, utilisés pour fabriquer des implants, tubulures, et accessoires, par exemple à usage urologique ou gynécologique. Cette adhérence a pour conséquence, sur le moyen et long terme, d'entraîner une résistance aux antibiotiques des souches et espèces bactériennes qui demeurent ainsi en permanence, et sous faible concentration, au contact des substances bactéricides administrées aux patients.

Grâce à l'invention, on obtient un biomatériau composite, présentant une excellente cohésion interne, en ce qu'il n'est pas susceptible en particulier de se délaminer, et ceci sans aucune opération ou intégration d'espèces chimiques susceptibles d'apporter une certaine toxicité vis-à-vis de milieux biologiques ou vivants, avec lesquels un objet ou matériau selon l'invention est susceptible d'entrer en contact.

La présente invention présente encore les modalités d'exécution suivantes.

Le matériau polymère, à l'état solide, a intrinsèquement, c'est-à-dire à l'état non lié avec le substrat de l'objet ou du matériau traité selon l'invention, une énergie de surface relativement importante, par exemple un angle de contact inférieur à 50° et par exemple compris entre 20° et 55°.

Le matériau polymère retenu selon l'invention est biocompatible, au sens où il peut être mis au contact d'un milieu biologique, par exemple un tissu vivant, sans affecter, c'est-à-dire sans modifier à court terme ce dernier. En particulier, cette biocompatibilité s'entend d'au moins l'une des propriétés suivantes, à savoir :
- quasi-absence de monomères ou oligomères
- pratiquement pas de traces de catalyseur de polymérisation
- pratiquement aucun additif, et/ou résidu de polymérisation.

Les groupements polaires retenus pour être greffés sur la surface exposée du substrat sont préférentiellement des groupements carboxy, hydroxy ou amino.

Le matériau polymère hydrosoluble est choisi préférentiellement dans le groupe constitué par :
- les celluloses et celluloses modifiées
- la polyvinylpyrrolidone et ses copolymères
- les polypropylèneglycols
- les polyacrylamides
- les polyacrylates
- les copolymères comprenant des motifs acrylate ou hydroxyéthylacrylate
- les alcools polyvinyliques
- les chitosans
- les polysaccharides.

A titre d'exemple, le matériau polymère hydrosoluble est une cellulose, ou une cellulose modifiée, choisie dans le groupe constitué par les hyroxypropylméthyl-celluloses (HPMC), les carboxyméthyl-celluloses, les méthylhydroxypropyl-celluloses, et les hydroxyéthyl-celluloses.

Avantageusement, le matériau polymère a une qualité pharmaceutique, c'est-à-dire est apte à entrer dans la composition ou formulation d'un médicament, et est donc par exemple répertorié en tant que tel dans la pharmacopée européenne et/ou américaine.

L'apport d'énergie in situ requis pendant l'étape (a) peut être effectué selon tout moyen approprié, par exemple au moyen de radiations gamma et ultra-violets, mais aussi par bombardement électronique.

Préférentiellement, cette étape (a) est effectuée en mettant la surface exposée du substrat, en contact avec un plasma comprenant l'espèce apte à former le ou les groupements polaires requis par l'invention.

S'agissant d'un plasma, les conditions particulières suivantes sont préférées :
- le plasma comprend de l'argon et est obtenu par apport in situ d'une énergie radio-fréquence, ayant une puissance comprise entre 3 et 10 W par litre d'atmosphère gazeuse, pendant une durée comprise entre environ 1 et 20 minutes
- ou le plasma est obtenu par apport in situ d'une énergie micro-onde, ayant une puissance comprise entre 3 et 10 W par litre d'atmosphère gazeuse, pendant une durée comprise entre 5 secondes et 20 minutes
- à titre d'exemple, le plasma est obtenu par apport d'énergie in situ, sous forme de décharges couronnes, avec une tension comprise entre 50 et 500 V, pendant une durée comprise entre 0,1 et 1 seconde ; cette décharge couronne peut être effectuée à l'aide d'un dispositif à électrodes parallèles en vis-à-vis, ou à électrodes parallèles côte à côte (avec un arc électrode d'environ 5 mm de hauteur), ou à arc soufflé (électrodes parallèles côte à côte avec un courant gazeux entre lesdites électrodes, créant ainsi arc électrique d'environ 10 cm de hauteur).

Si nécessaire, une étape d'oxydation de la surface exposée du substrat est effectuée, avant l'étape de greffage chimique, ou entre les étapes de greffage chimique et de couchage.

Par « groupement polaire », on entend tout groupement chimique susceptible de générer un ou plusieurs protons.

La présente invention concerne également un objet ou matériau, à savoir biomatériau, susceptible d'être obtenu par un procédé tel que défini précédemment. En particulier, l'invention concerne un objet ou matériau dont la surface exposée, par exemple extérieure, est susceptible d'entrer en contact avec un milieu biologique, tel que défini précédemment, cet objet ou matériau comprenant :
- un substrat dont une surface comprend des groupements polaires greffés chimiquement
- un matériau polymère déposé sur la surface greffée,
ce matériau étant caractérisé en ce que, en combinaison, d'une part le matériau polymère est hydrosoluble, et d'autre part ledit matériau polymère est lié, par liaison faible, par exemple par liaison hydrogène, à la surface greffée du substrat, et ce à l'exclusion de toute liaison chimique, d'une part entre le matériau polymère et la surface greffée exposée, et d'autre part au sein dudit matériau polymère, par réticulation.

Préférentiellement, les groupements polaires greffés pénètrent à partir de la surface du substrat, sur une épaisseur dudit substrat comprise entre 10 et 500 nm, et préférentiellement comprise entre 30 et 100 nm.

Le matériau polymère lié à la surface greffée du substrat a une épaisseur comprise entre 1 µm et 10 µm, et préférentiellement comprise entre 2 µm et 10 µm.

Comme dit plus haut, l'invention s'applique à des objets ou matériaux, dont les substrats sont les plus divers, et par exemple choisis parmi :
- les métaux, notamment acier et titane
- les céramiques
- les matières polymères naturelles ou synthétiques, thermoplastiques, thermo-durcissables, et visco-élastiques, par exemple caoutchouc.

Préférentiellement, le substrat d'un objet ou matériau selon l'invention est choisi dans le groupe constitué par le polyéthylène, les polysiloxanes, les polyamides, les polyéthers, les polyesters, et les polychlorures de vinyle.

Un biomatériau selon l'invention peut être utilisé pour la fabrication d'objet à usage médical, chirurgical, ou thérapeutique, par exemple à usage urinaire et gynécologique, avec les avantages décrits précédemment, résultant de leur faible adhérence biologique.

L'invention concerne aussi l'utilisation des matériaux précédemment décrits, en tant que matériaux anti-adhérence biologique.

La présente invention est maintenant décrite par référence à la partie expérimentale suivante.

Tout d'abord, le procédé de traitement comprend :
(a) une étape de greffage chimique, effectuée avec un plasma de type capacitif, dans un réacteur de 25 litres, sous émission radioélectrique de 10 à 400 W, et avec une durée de plusieurs minutes, et ce avec un matériel PLASMATECKNIK GRUN GmbH référence MPG
(b) l'étape de couchage est effectuée par simple immersion ou trempage du matériau ou objet greffé chimiquement, avec une solution aqueuse du matériau polymère, puis égouttage, et séchage sous hotte à flux laminaire pendant environ 1 heure.

L'objet testé consiste en une plaque d'un polyméthacrylate de méthyle (PMMA), ou polystyrène (PS), ou polyuréthanne (PU). Pour cet objet, l'étape (a) est effectuée avec un plasma d'argon, sous une puissance de décharge de 100 W, pendant une durée de 10 minutes.

Les matériaux polymères utilisés sont :
- PVP :: polyvinylpyrrolidone (grade pharmaceutique)
- HPMC :: hydroxypropylméthylcellulose E4M hydrosoluble (pharmaceutique)
- PAA :: polyacrylamide

Les solutions sont préparées avec de l'eau stérile (préparation injectable) et à des teneurs de :
1 % en PVP
0,5% en HPMC
1 % en PAA

Les échantillons de PMMA ayant été ainsi traités, la colonisation bactérienne de leurs surfaces exposées a été quantifiée au microscope à épifluorescence.

Le tableau 1 ci-après résume les résultats obtenus, selon les essais.

**TABLEAU 1**

| Substrat | Angle de contact avant traitement (°) | Conditions de traitement | Angle de contact après traitement (°) | Réf. |
|---|---|---|---|---|
| | 86 | Ar,100W, 10min, + HPMC 0,5% | 66 | PS tém. PS-HPMC |
| | | Ar,100W, 10min | 17 | PS-Ar |
| | | Ar,100W, 10min, + PVP 1% | 14 | PS-PVP |
| PMMA | 82 | Ar, 100W, 10min, + HPMC 0,5% | 52 | PMMA tem PMMA-HPMC |
| | | Ar,100W, 10min | 27 | PMMA-Ar |
| | | Ar,100W, 10min, + PVP 1% | 26 | PMMA-PVP |
| | | Ar,100W, 10min, + Polyvinlyalcool | 22 | PMMA-PVA |
| | | Ar,100W, 10min, + Polyacrylamide | 18 | PMMA-PAA |
| Ar : argon | | | | |

Les souches et conditions de culture suivantes sont utilisées :

La souche *d'Escherichia coli* PHL628 (référence : Isolation of an *Escherichia coli* K-12 Mutant Strain Able to Form Biofilms on Inert Surfaces : Involvement of a New ompR Allele That Increases Curli Expression, Olivier Vidal, Robert Longin, Claire Prigent-Combaret, Corinne Dorel, Michel Hooreman, and Philippe Lejeune, Journal of Bacteriology, Mai 1998, p 2442-2449) est capable de coloniser en quelques heures des surfaces telles que le verre et différents plastiques (PS, PMMA, PE, PP,...) grâce à la surproduction d'un type particulier de pili : les « curli ».

Les incubations de cette souche ont été effectuées à 30°C dans le milieu minimum M63G (H₂O ; KH₂ PO₄ ; (NH₄)₂SO₄ ; Fe₂(SO₄)₃ ; MgSO₄ ; vitamine B1 et glucose) en absence d'agitation.

Les cultures de la souche ont été ensemensées à DO₆₀₀ = 0,01.

Après différentes périodes d'immersion, les échantillons ont été lavés 2 fois dans du MgSO₄, 0.01 M (milieu isotonique avec le cytoplasme des bactéries), pour éliminer les bactéries libres. Les biofilms développés sur ces surfaces sont ensuite colorés au 4.6-diamidino-2-phénylindole (DAPI) et observés sous microscope à épifluorescence avec un grossissement de 625 et 1250 fois. Les observations ont été réalisées sur des champs d'environ 0,7 mm² (x1250) et 1,38 mm² (x 625).

Pour chaque échantillon, l'observation a été effectuée sur 25 champs d'observation situés sur 5 zones ou champs rectangulaires, dont l'un au centre, et les quatre autres dans les quatre angles respectivement.

Pour chaque essai, on mesure l'énergie de surface (angle de contact), et le nombre de bactéries par mm² (Nb/mm²), mesuré après immersion. Les résultats suivants sont obtenus.
**Substrat : PS** - PVP ; Ar ; HPMC

**4 heures d'immersion**

**TABLEAU 2**

| Support | Contact angle (°) | N_{b}/mm² |
|---|---|---|
| PS-HPMC | 66 | 5 |
| PS-Ar | 17 | 7 |
| PS-PVP | 14 | 59 |
| PS-tém. | 86 | 88 |

**6 heures d'immersion**

**TABLEAU 3**

| Support | Contact angle (°) | N_{b}/mm² |
|---|---|---|
| PS-HPMC | 66 | 26 |
| PS-Ar | 14 | 51 |
| PS-PVP | 86 | 251 |
| PS-tém. | 17 | 455 |

**Support : PMMA** - PVP ; Ar ; HPMC

**4 heures d'immersion**

**TABLEAU 4**

| Support | Contact angle (°) | N_{b}/mm² |
|---|---|---|
| PMMA-HPMC | 52 | 5 |
| PMMA-PVP | 26 | 40 |
| PMMA-Ar | 27 | 138 |
| PMMA-tém. | 83 | 145 |

**6 heures d'immersion**

**TABLEAU 5**

| Support | Contact angle (°) | N_{b}/mm² |
|---|---|---|
| PMMA-HPMC | 52 | 172 |
| PMMA-Ar | 27 | 232 |
| PMMA-tém. | 83 | 295 |
| PMMA-PVP | 26 | 451 |

**Support : PMMA** - PAA ; PVA ;

**6 heures d'immersion (1**^{**ère**} **expérience)**

**TABLEAU 6**

| Support | Contact angle (°) | N_{b}/mm² |
|---|---|---|
| PMMA-PVA | 22 | 2 |
| PMMA-PAA | 18 | 11 |
| PMMA-tém. | 83 | 184 |

**6 heures d'immersion (2ème expérience)**

**TABLEAU 7**

| Support | Contact angle (°) | N_{b}/mm² |
|---|---|---|
| PMMA-PVA | 22 | 10 |
| PMMA-PAA | 18 | 28 |
| PMMA-tém. | 83 | 55 |

**18 heures d'immersion**

**TABLEAU 8**

| | |
|---|---|
| PMMA tém | Surface recouverte de bactéries |
| PAA | Surface recouverte de bactéries |
| PVA | Bactéries associées en microcolonies épaisses et de grande taille |

**24 heures d'immersion**

**TABLEAU 9**

| | |
|---|---|
| PMMA tém | Surface entièrement recouverte par les bactéries, biofilm très épais |
| PAA | Surface recouverte par les bactéries, encore des surfaces vides |
| PVA | Comme PAA |

**Support : PMMA** - PVP ; Ar ; HPMC ; PVA

**6 heures d'immersion**

**TABLEAU 10**

| Support | Contact angle (°) | N_{b}/mm² |
|---|---|---|
| PMMA-PVA | 22 | 4 |
| PMMA-PVP | 26 | 4 |
| PMMA-HPMC | 52 | 10 |
| PMMA-PAA | 18 | 19 |
| PMMA-Ar | 27 | 114 |
| PMMA-tém. | 86 | 203 |

### Polyuréthanne (PU)

Les échantillons ont été immergés dans le milieu M63G pendant 4h, 6h, 8h, 15h, 18h, 21h et 24h. La colonisation bactérienne est nettement plus rapide avec le PU, par rapport aux autres types de surface. En effet, après 4h d'immersion, quelques microcolonies qui occupent la moitié de la surface de champ ont été observées. Au bout de 15h, la surface est bien colonisée.

La distribution des bactéries sur les surfaces est très irrégulière. Pendant les premières heures d'immersion, sur une même surface, on peut trouver soit des bactéries isolées en nombre variable, soit des microcolonies de taille variable.

Il y a une limitation de la colonisation bactérienne avec certains types de molécules comme :
HPMC (hydroxypropylméthyl cellulose) : après 4h et 6h d'immersion (le résultat est reproductif dans tous les essais).
PVP (polyvinylpyrrolindone) : après 4h et 6h d'immersion.
PVA (polyvinylalcool), PAA (polyacrylamide).

D'autres essais ont encore été effectués à partir de la même souche PHL *d'Escherichi coli,* à savoir PHL628, et d'une autre souche de cette même bactérie, à savoir HH97531012 (cf. publication Vidal et al, précitée), isolée à partir de l'urine d'un malade porteur d'une sonde urinaire contaminée, et ce en se rapprochant des conditions naturelles de contamination des biomatériaux, à savoir avec une faible densité bactérienne.

Les tableaux ci-après N°11 à 13 résument les conditions de ces essais et les résultats obtenus, avec le milieu M63G.

Les incubations de ces deux souches ont été effectuées à 30°C dans le milieu minimum M63G (H₂O ; KH₂PO₄; (NH₄)₂SO₄; Fe₂(SO₄)₃; MgSO₄; vitamine B1 et glucose) et dans l'urine (SIGMA) en absence d'agitation.

La densité bactérienne est d'environ 300 bact./ml (DO₆₀₀ = 8.10⁻⁷).

Après 24h et 48h d'immersion, les échantillons ont été lavés 2 fois dans du MgSO₄ 0.01M (milieu isotonique avec le cytoplasme des bactéries), pour éliminer les bactéries libres. Les bactéries fixées sur ces surfaces sont ensuite fixées au paraformaldéhyde 4% pendant au moins 4 heures, puis colorées au 4.6-diamidino-2-phénylindole (DAPI) (2µg/ml, 30min), puis lavées 2 fois dans l'eau distillée et enfin recouvertes d'une goutte de glycérol 80%.

Les observations microscopiques ont été effectuées en immersion. Les observations ont été réalisées sur des champs d'environ 1,38 mm² (x625), et dans les mêmes conditions que celles décrites précédemment.
Souche *d'Escherichia coli* K-12 (PHL628)

**24 heures d'immersion**

**TABLEAU 11**

| Substrat | Angle de contact (°) | Nb/mm² |
|---|---|---|
| PMMA-PAA | 83 | 3 |
| PMMA-PVP | 14 | 6 |
| PMMA-PVA | 26 | 6 |
| PMMA-HPMC | 66 | 9 |
| PMMA-Ar | 17 | 18 |
| PMMAtém. | 86 | 32 |

**48 heures d'immersion**

**TABLEAU 12**

| Substrat | Angle de contact (°) | Nb/mm² |
|---|---|---|
| PMMA-PVP | 14 | 1 |
| PMMA-PAA | 83 | 1 |
| PMMA-PVA | 26 | 2 |
| PMMA-HPMC | 66 | 3 |
| PMMA-Ar | 17 | 10 |
| PMMAtém. | 86 | 77 |

Souche clinique *d'Escherichia coli* (HH97531012)

**24 heures d'immersion**

**TABLEAU 13**

| Substrat | Angle de contact (°) | Nb/mm² |
|---|---|---|
| PMMA-PAA | 83 | 1 |
| PMM-PVP | 14 | 2 |
| PMMA-PVA | 26 | 4 |
| PMMA-HPMC | 66 | 6 |
| PMMA-tém. | 86 | 48 |
| PMMAt-Ar | 17 | 86 |

**48 heures d'immersion**

Les surfaces sont entièrement recouvertes par les bactéries

Immersion dans l'urine

Isolat clinique d'Escherichia coli (HH97531012)

**24 heures d'immersion**

**TABLEAU 14**

| | |
|---|---|
| PMMA tém | Biofilm très épais (épaisseur de 10 à 20 µm) |
| PMMA-Ar | Surface recouverte d'une monocouche de bactéries (épaisseur : environ 1 µm) |
| PMMA-PVP | Bactéries dispersées en microcolonies à monocouches et de taille variable |
| PMMA-HPMC | Bactéries dispersées en microcolonies à monocouche, plus petites que sur le PVP |
| PMMA-PVA | Bactéries soit isolées, soit dispersées en petites microcolonies à monocouche |
| PMMA-PAA | Comme PVA |

**48 heures d'immersion**

Les surfaces sont entièrement recouvertes de bactéries sauf dans le cas de PMMA-HPMC, on observe des bactéries isolées ou quelques conglomérats de bactéries séparés par de grands espaces vides.

Les objets ou matériaux obtenus selon la présente invention ont un vaste champ d'applications, en raison de leur faible adhérence biologique, c'est-à-dire de leur aptitude à se lier à des micro-organismes, principalement bactéries.

A ce titre, ils constituent des biomatériaux de choix, et sont particulièrement bien adaptés pour la réalisation, l'obtention, et la fabrication d'objets, équipements, ou pièces à usage médical ou chirurgical, dans toutes sortes d'indications ou applications, par exemple pour constituer des implants, tubulures et accessoires en milieu urologique et gynécologique.

## Revendications

1. Utilisation en tant qu'objet ou matériau dont la surface extérieure est anti-adhérente vis à vis de micro-organismes, par exemple des bactéries, et/ou vis à vis d'entités biologiques comportant des protéines, d'un objet ou matériau susceptible d'être obtenu par le procédé suivant :
a) On part d'undit objet ou matériau comprenant un substrat ayant une surface exposée ;
b) On effectue un greffage chimique en une seule étape pendant laquelle concomitamment, d'une part on met la surface exposée dudit objet ou matériau, en contact avec une atmosphère gazeuse comprenant au moins une espèce chimique, susceptible d'être liée chimiquement audit substrat au niveau de sa surface exposée, sans se polymériser, et apte à former un groupement polaire une fois liée audit substrat, et d'autre part on active l'espèce chimique et/ou la surface exposée, par apport d'énergie in situ, par exemple rayonnée ;
c) Selon une étape de couchage, on dépose au moins un matériau polymère hydrosoluble sur la surface exposée greffée, et ledit polymère hydrosoluble est lié par liaison faible, par exemple par liaison hydrogène, à la surface exposée greffée, à l'exclusion de pratiquement toute liaison chimique, d'une part entre ledit matériau polymère et la surface greffée exposée, et d'autre part au sein dudit matériau polymère, par réticulation.

2. Utilisation selon la revendication 1, caractérisée en ce que le matériau polymère, à l'état solide, a intrinsèquement une énergie de surface relativement importante, par exemple un angle de contact inférieur à 55°, et par exemple compris entre 20° et 55°.

3. Utilisation selon la revendication 1, caractérisée en ce que le matériau polymère est biocompatible, au sens où il peut être mis au contact d'un milieu biologique, par exemple un tissu vivant, sans affecter à court terme ce dernier.

4. Utilisation selon la revendication 3, caractérisée en ce que la biocompatibilité du matériau polymère s'entend d'au moins l'une des propriétés suivantes, à savoir :
- quasi-absence de monomères ou oligomères,
- pratiquement pas de traces de catalyseur de polymérisation,
- pratiquement aucun additif, et/ou résidu de polymérisation.

5. Utilisation selon la revendication 1, caractérisée en ce que les groupements polaires sont choisis dans le groupe constitué par les groupements carboxy, hydroxy et amino.

6. Utilisation selon la revendication 1, caractérisée en ce que le matériau polymère est choisi dans le groupe constitué par :
- les celluloses et celluloses modifiées,
- la polyvinylpyrrolidone et ses copolymères,
- les polypropylèneglycols,
- les polyacrylamides,
- les polyacrylates,
- les copolymères comprenant des motifs acrylate ou hydroxyéthylacrylate
- les alcools polyvinyliques,
- les chitosans,
- les polysaccharides.

7. Utilisation selon la revendication 6, caractérisée en ce que le matériau polymère est une cellulose ou une cellulose modifiée, choisie dans le groupe constitué par les hydroxypropylméthyl-celluloses (HPMC), les carboxyméthyl-celluloses, les méthylhydroxypropyl-celluloses, et les hydroxyéthyl-celluloses.

8. Utilisation selon la revendication 6, caractérisée en ce que le matériau polymère a une qualité pharmaceutique, c'est-à-dire est apte à entrer dans la composition ou formulation d'un médicament.

9. Utilisation selon la revendication 1, caractérisée en ce que l'étape a) est effectuée en mettant la surface exposée en contact avec un plasma comprenant ladite espèce chimique.

10. Utilisation selon la revendication 9, caractérisée en ce que le plasma comprend de l'argon et est obtenu par apport in situ d'une énergie radio-fréquence, ayant une puissance comprise entre 3 et 10 W par litre d'atmosphère gazeuse, pendant une durée comprise entre environ 1 et 20 minutes.

11. Utilisation selon la revendication 9, caractérisée en ce que le plasma est obtenu par apport in situ d'une énergie micro-onde, ayant une puissance comprise entre 3 et 10 W par litre d'atmosphère gazeuse, pendant une durée comprise entre 5 secondes et 20 minutes.

12. Utilisation selon la revendication 11, caractérisée en ce que le plasma est obtenu par apport d'énergie in situ, sous forme de décharges couronnes, avec une tension comprise entre 50 et 500 V, pendant une durée comprise entre 0,1 et 1 seconde.

13. Utilisation selon la revendication 1, caractérisée en ce que l'étape b) est effectuée par trempage du substrat résultant de l'étape a) de greffage chimique, dans une solution aqueuse du matériau polymère, puis séchage.

14. Utilisation selon la revendication 1, caractérisée en ce qu'une étape d'oxydation de la surface exposée est effectuée, avant l'étape a), ou entre les étapes a) et b).

15. Utilisation selon la revendication 1, caractérisée en ce que les groupements polaires greffés pénètrent à partir de la surface du substrat, sur une épaisseur comprise entre 10 et 500 nm, et préférentiellement comprise entre 30 et 100 nm.

16. Utilisation selon la revendication 1, caractérisée en ce que le matériau polymère lié à la surface greffée du substrat a une épaisseur comprise entre 1 µm et 10 µm, et préférentiellement comprise entre 2 µm et 10 µm.

17. Utilisation selon la revendication 1, caractérisée en ce que le substrat est choisi dans le groupe constitué par :
- les métaux, notamment acier et titane,
- les céramiques,
- les matières polymères naturelles ou synthétiques, thermoplastiques, thermo-durcissables, et visco-élastiques.

18. Utilisation selon la revendication 17, caractérisée en ce que le substrat est choisi dans le groupe constitué par le polyéthylène, les polysiloxanes, les polyamides, les polyéthers, les polyesters, et les polychlorures de vinyle.

19. Utilisation selon la revendication 1 caractérisée en ce que l'objet est à usage médical, chirurgical, ou thérapeutique, par exemple à usage urinaire et gynécologique.
